# EUROPEAN PATENT SPECIFICATION

(11) **EP 2 803 352 B1**
(45) Date of publication and mention of the grant of the patent: **01.08.2018**
(21) Application number: 14001695.7
(22) Date of filing: 14.05.2014
(51) Int. Cl.: A61K 9/20, A61K 9/28, A61K 31/506

(54) **High dose imatinib tablets**
Zusammensetzungen von Imatinib
Compositions d'imatinib

(30) Priority: 14.05.2013 IN CH21372013
(43) Date of publication of application: 19.11.2014
(73) Proprietor: Hetero Research Foundation, Hyderabad 500 018 (IN)
(72) Inventor: Parthasaradhi Reddy, Bandi, Balanagar Hyderabad Telangana 500018 (IN); Khadgapathi, Podili, Jeedimetla Hyderabad Telangana 500055 (IN); Kamalakar Reddy, Goli, Jeedimetla Hyderabad Telangana 500055 (IN); Kiran Kumar, Madallapalli, Jeedimetla Hyderabad Telangana 500055 (IN)
(74) Representative: Kernebeck, Thomas

(56) References cited:
- WO-A2-2012/087255
- WO-A2-2013/008253
- ZARIANA NIKOLOVA ET AL: "Bioequivalence, safety, and tolerability of imatinib tablets compared with capsules", CANCER CHEMOTHERAPY AND PHARMACOLOGY, vol. 53, no. 5, 1 May 2004 (2004-05-01), pages 433-438, XP055128465, ISSN: 0344-5704, DOI: 10.1007/s00280-003-0756-z
- Anonymous: "Calcium Phosphate, Tribasic: Pharmaceutical Excipients", Handbook of Pharmaceutical Excipients, 6 April 2017 (2017-04-06), pages 1-5, XP055402876, Retrieved from the Internet: URL:https://www.medicinescomplete.com/mc/e xcipients/current/1001934973.htm?q=calcium phosphate&t=search&ss=text&tot=67&p=1#_hit [retrieved on 2017-08-31]
- Raymond C. Rowe Et Al: "Povidone" In: "Handbook of Pharmaceutical Excipients", 1 January 2000 (2000-01-01), Pharmaceutical Press [u.a.], London [u.a.] 031835, XP055396598, ISBN: 978-1-58212-058-4 pages 611-616,

## Description

The present invention relates to pharmaceutically acceptable compositions comprising imatinib, more specifically high dose film-coated tablets comprising imatinib mesylate.

### BACKGROUND ART

Imatinib is a tyrosine-kinase inhibitor used in the treatment of certain types of cancers. Imatinib mesylate is designated chemically as 4-[(4-Methyl-1-piperazinyl)methyl]-N-[4-methyl-3-[[4-(3-pyridinyl)-2-pyrimidinyl]amino]-phenyl]benzamide methanesulfonate. It has the following molecular structure:

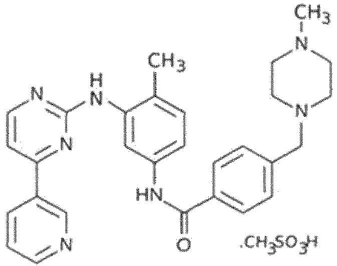

Imatinib as well as processes for its preparation are disclosed in European patent application EP0564409A.

Imatinib is marketed by Novartis under the trademark Glivec® in Europe. It is indicated for treatment of Philadelphia chromosome positive acute lymphoblastic leukaemia (Ph-positive ALL), Myelodysplastic/myeloproliferative diseases (MDS/MPD), Hypereosinophilic syndrome (HES) and/or chronic eosinophilic leukaemia (CEL), Gastrointestinal stromal tumours (GIST), Dermatofibrosarcoma protuberans (DFSP).

Currently, Glivec® dosage forms authorized by European Medicines Agency comprise imatinib in the form of its mesylate salt and in dosages equivalent to 50 mg and 100 mg of imatinib free base as hard capsules and of 100 mg and 400 mg as film-coated tablets.

According to Glivec® SmPC, imatinib doses of 400 mg or 600 mg should be administered once daily, whereas a daily dose of 800 mg should be administered as 400 mg twice a day, in the morning and in the evening.

Thus, currently a patient that needs to take a dose of 600 mg of imatinib needs to take several tablets, namely three pills (one of 400 mg and two of 100 mg) or one of 400 mg and a half of a 400 mg.

It is well known that dysphagia (swallowing dysfunction) and odynophagia (painful swallowing) are common in cancer patients. For patients unable to swallow the tablets or capsules, according to Glivec® SmPC, the compositions may be dispersed in a glass of mineral water. However, the required number of tablets should be placed in the appropriate volume of beverage (approximately 50 ml for a 100 mg tablet, and 200 ml for a 400 mg tablet), so a dose of 600 mg would require as much as 300 ml. Further, special warnings with handling of capsules are highlighted due to reproductive toxicity of content shown in animals.

Lack of adherence to oral therapy for chronic diseases is a well-recognized problem. Several studies have shown that lack of adherence to imatinib treatment is frequent and has a significant impact on the degree of response to therapy obtained by the patient. Recent reports conclude that poor adherence to imatinib therapy is the most important factor contributing to cytogenetic relapse and imatinib failure. Said reports highlight that it is of paramount importance that physicians recognize this problem and work toward improving adherence in chronic myeloid leukemia patients treated with tyrosine kinase inhibitors (cf. Amr R. Ibrahim et al., 'Poor adherence is the main reason for loss of CCyR and imatinib failure for chronic myeloid leukemia patients on long-term therapy' Blood, 2011 vol. 117, no. 14, pgs. 3733-3736).

Thus, any attempt that could result in improving patient adherence to imatinib therapy would be highly desirable.

Pharmaceutical dosage forms with high active ingredient content pose a challenge for pharmaceutical development. Large doses may be a problem for direct compression. Few references mention generically high loaded imatinib pharmaceutical dosage forms (i.e. with a dose higher than 400 mg) and specifically disclosed examples require high volume capsules.

WO2012080703 by CiplaLtd. discloses imatinib capsules comprising more than 100 mg of imatinib base, in particular it provides examples of capsules of imatinib mesylate corresponding to 100 mg, 200 mg, 400 mg and 800 mg of imatinib base. For this last dose, capsule of size 000 (26 mm length) is employed. The content of imatinib base in the capsules is not more than 80% (around 79%). The imatinib is wet granulated with isopropyl alcohol and purified water and thereafter mixed with crospovidone and magnesium stearate. Thus, it does not teach how to manufacture high content imatinib tablets with convenient size.

US2007036850A1 by Siegfried Generics International AG, discloses imatinib granules or film-coated tablets obtained by dry granulation of the active ingredient together with one filler-binder. The application mentions that the content of active ingredient may be from 25-80%, preferably 50-70%, and that the dosage unit may contain 100, 200, 300, 400 or 600 mg. However, it only provides examples of tablets comprising 100 mg and 400 mg of imatinib base, wherein imatinib base constitutes around 50% of the total weight of the tablet and comprise 36% of excipients with regard to total weight of tablet core. In fact, as US2007036850A1 discloses in paragraph [0026] and [0027], preferred concentrations of filler-binders are those ranging from 75% by weight to 20% by weight, until the most preferred of 50% by weight to 30% by weight. Moreover, in paragraph [0027] the same reference states that when other optional additives are present in the composition, it is advantageous to correspondingly reduce the proportion by weight of filler-binder.

Thus, it does not teach how to manufacture high content imatinib tablets with convenient size.

EP2497464, by Adamed SP., discloses imatinib capsules prepared by dry granulation of imatinib mesylate alpha form. The blend for filling unit dosage form is adjusted so that the content of imatinib methanesulfonate in a unit dosage corresponds to 50, 100, 200, 300, 400 or 600 mg of imatinib free base. For a dose of 400 mg capsules of size 00 (total length: 23.3 mm, maximum diameter: 8.53 mm) are employed. So, for higher doses e.g. 600 mg, even a bigger capsule (e.g. 000 of 26 mm length) is expected to be needed. Further, it mentions that when performing experiments it was observed that subjecting a mixture containing imatinib methanesulfonate crystalline alpha form to repeated compactions may increase the risk of partial conversion of this metastable form into a thermodynamically more stable form and that similar risks would be also bound with the use of solvents during the production of a composition.

Thus, it does not teach how to manufacture high dose imatinib tablets with convenient size.

The prior art includes examples of relatively high loaded tablets of imatinib mesylate, although they are silent about the manufacture of dosings greater than 400 mg. For instance, WO2011160798 by Polpharma discloses a tablet of imatinib mesylate which does not include any binder in the core. However, WO2011160798 is silent about manufacturing a high dose imatinib tablet with convenient size.

WO2012087255 discloses compositions comprising imatinib and a pharmaceutically acceptable disintegrant in the range of 0.1% to 5% by weight and at least one other excipient.

Additionally, stability is also of critical importance when developing pharmaceutical dosage forms with high active ingredient dosage. The present inventors have observed that prior art imatinib high loaded tablet compositions show stability problems when subjected to stress conditions, e.g. polymorphic conversion issues.

Several crystalline forms of imatinib mesylate have been disclosed in the art, some of them with known stability issues. International application WO9903854 discloses alpha and beta crystalline forms of imatinib mesylate, characterized among others by characteristic X-ray powder diffraction patterns. International application WO2005095379 discloses crystalline forms I and II of imatinib dimesylate. International application WO2004106326 discloses crystalline form H1, WO2007023182 forms delta and epsilon, and WO2007059963 forms F, G, H, I and K of imatinib mesylate.

Therefore, there is the need in the prior art for providing imatinib compositions which allow improving patient compliance, in particular when high doses are needed. Additionally, there is a need to develop high drug loaded tablets of imatinib with high stability, particularly which are stable under stress conditions, e.g. stable against polymorphic conversion.

### SUMMARY

The present invention provides high dose, film-coated tablet dosage forms for immediate release consisting of imatinib mesylate in an amount equivalent to 600 mg of imatinib free base and at least one pharmaceutically acceptable excipient as defined in the first aspect of the invention which are convenient for improving patient compliance, more advantadgeously in treatments including high doses of imatinib. Additionally, high dose tablets of the invention comprise a high percentage of the active ingredient obtaining smaller tablets which are easier to swallow, further helping to improve patient compliance. The compositions of the invention have shown other improvements over prior art compositions, for instance, the inventors have found that the compositions of the invention are stable and prevent imatinib mesylate from undergoing polymorphic changes upon tabletting process and storage.

Therefore, the first aspect of the present invention is directed to an immediate release oral pharmaceutical composition comprising consisting of imatinib mesylate in an amount equivalent to 600 mg of imatinib free base and at least one pharmaceutically acceptable excipient, wherein the composition is in the form of a film-coated tablet comprising from 95% to 99%by weight of imatinib mesylate salt based on the total weight of the film-coated tablet wherein the pharmaceutically acceptable excipients comprised in the tablet core are selected from the group consisting of diluents, glidants, lubricants, and combinations thereof wherein:
diluents are selected from the group consisting of lactose, calcium carbonate, calcium sulfate, sugar, dextrates, dextrin, dextrose, dibasic calcium phosphate, kaolin, magnesium carbonate, magnesium oxide, maltodextrin, mannitol, polymethacrylates, potassium chloride, sodium chloride, sorbitol, talc, and combinations thereof;
glidants are selected from the group consisting of magnesium trisilicate, talc, and combinations thereof; and
lubricants/lubricating agents are selected from the group consisting of magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, zinc stearate, and combinations thereof.

The second aspect of the invention is directed to a process for preparing the oral pharmaceutical composition of the invention comprising mixing the active ingredient with at least one pharmaceutically acceptable excipients.

### DETAILED DESCRIPTION OF THE INVENTION

An immediate-release dosage form, as defined in European Pharmacopoeia 7.0 04/2010:1502, is a preparation showing a release of the active substance which is not deliberately modified by a special formulation design and/or manufacturing method. In the case of a solid dosage form, the dissolution profile of the active substance depends essentially on its intrinsic properties.

In a preferred embodiment of the invention, the pharmaceutical composition comprises from 96.77% to 99% by weight of imatinib mesylate salt based on total weight of the film-coated tablet.

For the purposes of the present invention, unless otherwise stated, the term "percentage (%) by weight" refers to the percentage of each ingredient of the composition in relation to the total weight of the final film-coated tablet.

According to the present invention, the composition comprises a tablet core and an immediate release film-coating. The tablet core may comprise pharmaceutically acceptable excipients selected from diluents, glidants, lubricants and combinations thereof.

The expression "pharmaceutically acceptable excipients" or simply "excipients" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The diluents are selected from the group consisting of lactose, calcium carbonate, calcium sulfate, sugar, dextrates, dextrin, dextrose, dibasic calcium phosphate, kaolin, magnesium carbonate, magnesium oxide, maltodextrin, mannitol, polymethacrylates, potassium chloride, sodium chloride, sorbitol, talc, and combinations thereof.

The glidants are selected from the group consisting of magnesium trisilicate, talc, and combinations thereof.

The lubricants/lubricating agents are selected from the group consisting of magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, zinc stearate, and combinations thereof. Preferred lubricant is magnesium stearate.

According to the invention, the tablet core does not comprise any disintegrant.

In a preferred embodiment, the tablet core of the compositions of the invention consists of imatinib mesylate and one or more lubricants, more preferably magnesium stearate.

In a prefered embodiment of the invention, the tablet core is manufactured by direct compression.

Advantageously, the invention provide small tablets with a high load of drug, maintaining the amount of excipients as low as possible while meeting the requirements of bioavailability and bioequivalence of pharmaceutical products.

The immediate release film coating of the composition according the present invention may comprise any pharmaceutically acceptable film-coating agent suitable for the immediate release of the active pharmaceutical ingredient, for example, polyvinyl alcohol, cellulose derivatives (such as hydroxypropyl methyl cellulose, hydroxypropyl cellulose, hydroxyethyl cellulose, ethyl cellulose, and methyl cellulose), polymethacrylates (such as Eudragit RS), polyvinylpyrrolidone, polyvinyl alcohol, and mixtures thereof.

In a particular embodiment, the immediate release film coating comprises hydroxypropyl methylcellulose or polyvinyl alcohol.

In a preferred embodiment, the composition of the invention comprises an immediate release film coating agent comprising hydroxypropylmethyl cellulose, for example hydroxypropyl methylcellulose based Opadry® product range and mixtures of hydroxypropyl methylcellulose and polyethylene glycol. More particularly, the film coating agent is an Opadry® composition comprising hydroxypropyl methylcellulose, polyethylene glycol/macrogol and talc.

Preferably, the composition of the invention is coated with a solution or dispersion in an organic solvent or mixture of organic solvents of a film coating agent comprising hydroxypropylmethyl cellulose.

By an organic solvent based film coating is meant herein a film coating that is applied employing a coating solvent system consisting of an organic solvent or mixture of organic solvents.

Organic solvent or mixture of organic solvents as described herein may contain up to a 10% (w/w) of water, preferably the solvents are anhydrous. By anhydrous organic solvent it is mean herein an organic solvent that contain less than 5% (w/w) of water, more preferably 0.5% or less.

In a particular embodiment, the organic solvent or mixture of organic solvents is selected from C1-C3 alcohol, e.g. methanol, ethanol or isopropyl alcohol, ethyl acetate, ethyl lactate, acetone, methylenechloride, 1,1,1-trichloroethane, chloroform, and mixtures thereof. Particularly, the solvent system is a mixture of C1-C3 alcohol/methylene chloride, more particularly Isopropyl alcohol/methylene chloride.

Imatinib mesylate has been reported to exist in several crystalline plymorphic forms as well as in amorphous form (e.g. WO9903854). The composition of the invention may comprise imatinib mesylate in crystalline or amorphous forms. Preferably, the imatinib mesylate is in alpha form or in amorphous form, more preferably in alpha form.

In a prefered embodiment of the invention,the immediate release film coated tablet of the invention preferably comprises imatinib mesylate in alpha crystalline form, characterized by a powder X-ray diffraction (PXRD) pattern having peaks at following 2θ angle positions: about 4.9, 10.5, 14.9, 16.5, 17.7, 18.1, 18.6, 19.1, 21.3, 21.6, 23.2, 24.9, 28.0, and 28.6 ± 0.2 degrees.

In a prefered embodiment of the invention, the immediate release film coated tablet of the invention is coated with a solution or dispersion of a film coating agent in an organic solvent or a mixture of organic solvents, and comprises imatinib mesylate in alpha crystalline form. Advantadgeously, imatinib salt does not undergo polymorphic conversion in said immediate release film coated tablet upon storage under stress conditions. In particular, in said immediate release film coated tablet, said alpha crystalline form of imatinib mesylate is retained upon storage at 40°C and at 75% relative humidity for 3 months, when measured by XRPD technique.

The crystalline form alpha of imatinib mesylate is a polymorphic form as prepared and characterized by methods disclosed in WO9903854, more specifically as shown in Example 1 of WO9903854.

Polymorphic conversion is measured by techniques known in the art. Preferably, the percentage of polymorphic conversion is measured by known PXRD techniques. For instance, in a mixture of polymorphs the amount of each polymorph can be calculated with reference to the relative intensity of the unique characterizing XRD peaks of each polymorph.

Additionally disclosed is a stable film-coated immediate-release tablet composition comprising alpha crystalline form of imatinib mesylate in an amount equivalent to 600 mg of imatinib free base and at least one pharmaceutically acceptable excipient, wherein the tablet composition retains its initial polymorphic form upon storage (e.g. during 1 to 3 months at 40°C±2°C and 75%±5% relative humidity). More specifically, wherein said film coating is applied as an organic solvent based film coating comprising hydroxypropyl methylcellulose.

In a particular disclosure, the immediate-release film-coated tablet retains alpha crystalline form of imatinib mesylate upon storage at 40°C, 75% RH, for 3 months.

In a further preferred embodiment the pharmaceutical composition of the invention consists of 95%-99% of imatinib mesylate (a-form), 0-3% of lactose monohydrate, 0.1%-1% of magnesium stearate, and 0.75%-1.25% of a film coating agent comprising hydroxypropylmethyl cellulose (Opadry), based on the total weight of the film coated tablet. In a particular embodiment the film coating agent comprising hydroxypropylmethyl cellulose (Opadry) is applied with an organic solvent.

In an alternative particular embodiment, the composition consists of 95%-99% of Imatinib mesylate (a-form), 0.5%-2% of Magnesium stearate, and 0.2%-3% of a film coating agent comprising hydroxypropylmethyl cellulose (Opadry).

According to the second aspect, the invention encompasses a process for preparing the oral pharmaceutical composition of the invention comprising mixing the active ingredient with at least one pharmaceutically acceptable excipients.

More particularly, tablet core compositions of the present invention can be prepared according to methods well known in the state of the art. Most preferred method for preparing the tablet core of the compositions of the invention is by direct compression.

In an embodiment of the process of the invention, the tablet core is prepared by direct compression of imatinib mesylate, together with one or more pharmaceutically acceptable excipients or carriers for preparing the tablet core. In another embodiment, the tablet core is prepared by direct compression of imatinib mesylate, a lubricant and optionally one or more excipients. Preferably imatinib is in alpha form.

In an embodiment of the invention, the process for the preparation of the pharmaceutical composition of the invention comprises mixing imatinib mesylate with a lubricant followed by direct compression of the resulting mixture.

Direct compression involves blending imatinib mesylate as crystalline alpha form and one or more excipients and compressing it directly into a tablet. Direct compression is easy, simple and applicable for industrial scale. The appropriate conditions and/or equipment used for suitable compressing the blend for obtaining the core tablet can readily be determined by those skilled in the art according to the tablet to be prepared.

The coating step of the tablet core is carried out using an organic solvent or a mixture of organic solvents. Preferably, the process of the invention comprises the step of coating the tablet core with a solution or dispersion in an organic solvent or mixture of organic solvents of a film coating agent comprising hydroxypropylmethyl cellulose.

In a particular embodiment, the organic solvent or mixture of organic solvents used in the process of the invention is selected from C1-C3 alcohol, e.g. methanol, ethanol or isopropyl alcohol, ethyl acetate, ethyl lactate, acetone, methylenechloride, 1,1,1-trichloroethane, chloroform, and mixtures thereof. Particularly, the solvent system is a mixture of C1-C3 alcohol/methylene chloride, more particularly Isopropyl alcohol/methylene chloride.

In an embodiment of the invention, the solvent system of the coating step is anhydrous, i.e. it has a content of water up to 5%, preferably less than 0.5% in weight of the solvent system.

The coating step of the process of the invention encompasses applying the coating solution to the core tablet and drying the coated tablet for removing the solvent and obtaining the final film-coated tablet by processes and methods known in the art. The appropriate conditions and/or equipment used, can readily be determined by those skilled in the art according to the tablet being prepared.

With regard to the specific conditions for carrying out the processes of the invention, the skilled person would know how to adjust the parameters of each of the steps indicated above in the light of the description and examples of the present invention.

Advantageously, the process of the invention provides small tablets with a high load of drug, maintaining the amount of excipients as low as possible while meeting the requirements of bioavailability and bioequivalence of pharmaceutical products.

The immediate-release film-coated tablet of the invention has surprisingly shown improved storage stability maintaining imatinib mesylate stable in crystalline alpha form without undergoing polymorphic conversion upon tabletting process and/or storage.

Polymorphic conversion is measured by techniques known in the art. Preferably, the percentage of polymorphic conversion is measured by known PXRD techniques. For instance, in a mixture of polymorphs the amount of each polymorph can be calculated with reference to the relative intensity of the unique characterizing XRD peaks of each polymorph.

In yet further embodiment of the invention is directed to a composition comprising a tablet core comprises 95% to 99% of imatinib mesylate based on total weight of the film coated tablet composition in a total amount equivalent to 600 mg of imatinib free base and one or more excipients, wherein the pharmaceutically acceptable excipients comprised in the tablet core are selected from the group consisting of diluents, glidants, lubricants, and combinations thereof wherein:
diluents are selected from the group consisting of lactose, calcium carbonate, calcium sulfate, sugar, dextrates, dextrin, dextrose, dibasic calcium phosphate, kaolin, magnesium carbonate, magnesium oxide, maltodextrin, mannitol, polymethacrylates, potassium chloride, sodium chloride, sorbitol, talc, and combinations thereof; glidants are selected from the group consisting of magnesium trisilicate, talc, and combinations thereof; and
lubricants/lubricating agents are selected from the group consisting of magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, zinc stearate, and combinations thereof;
and wherein the tablet core is coated with an organic solvent film coating solution comprising hydroxypropyl methylcellulose.
Additionally disclosed is a stable film-coated immediate-release tablet composition comprising alpha crystalline form of imatinib in an amount equivalent to 600 mg of imatinib free base and a pharmaceutically acceptable excipient, wherein the tablet composition retains its initial polymorphic form upon storage (e.g. during 1 to 3 months at 40°C±2°C and 75%±5% relative humidity). More specifically, wherein said film coating is applied as an organic solvent based film coating comprising hydroxypropyl methylcellulose.

A further aspect of the invention is directed to a stable film coated tablet composition comprising, based on total weight of the film coated tablet, 97 to 99% of alpha crystalline form of imatinib mesylate composition in a total amount equivalent to 600 mg of imatinib free base, 0.1 to 1% of magnesium stearate and 0.75 to 1.25% of hydroxypropyl methylcellulose based film coating; and the composition is prepared by direct compression process. Preferably, wherein the film coating is an organic solvent based film coating.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### Examples 1 to 5

| **Ingredients (mg/tablet)** | **Example 1** | **Example 2** | **Reference Example 3** | **Reference Example 4** | **Reference Example 5** |
|---|---|---|---|---|---|
| Imatinib mesylate (α-form) | 717^{#} | 717^{#} | 717^{#} | 717^{#} | 717^{#} |
| Lactose monohydrate | -- | -- | 4.00 | 16.00 | - |
| Microcrystalline cellulose | -- | -- | 6.50 | - | 10.50 |
| Povidone | -- | -- | 2.00 | 2.00 | - |
| Magnesium stearate | 6.00 | 4.00 | 4.50 | 4.50 | 4.50 |

| Coating: | | | | | |
|---|---|---|---|---|---|
| Opadry® yellow 03F82597* | 7.23 | 7.17 | 7.215 | 7.215 | 7.215 |
| Isopropyl alcohol | q.s. | q.s. | q.s. | q.s. | q.s. |
| Methylene chloride | q.s. | q.s. | q.s. | q.s. | q.s. |
| Coated tablet weight | 730.23 | 728.170 | 741.215 | 746.715 | 739.215 |
| **API% wrt coated tablet** | **98.19** | **98.46** | **96.73** | **96.02** | **96.99** |

| | | | | | |
|---|---|---|---|---|---|
| #Each 717mg of Imatinib mesylate contains 600mg of Imatinib. *Opadry® yellow 03F82597 composition comprises hydroxypropyl methylcellulose, iron oxide yellow, polyethylene glycol/macrogol, talc, titanium dioxide and iron oxide red. | | | | | |

### Brief manufacturing process:

1. All the ingredients except magnesium stearate were sifted and loaded into blender.
2. Magnesium stearate was sifted.
3. Blend of step 1, was lubricated with magnesium stearate of step 2.
4. Lubricated blend of step 3, was compressed into tablet.
5. Tablet of step 4, was film coated using Opadry® yellow 03F82597 dispersion in isopropyl alcohol and methylene chloride.

### REFERENCES CITED IN THE APPLICATION

### Patent Documents

- EP0564409A
- WO199903854
- WO2005095379
- WO2004106326
- WO2007023182
- WO2007059963
- WO2012080703
- US2007036850A1
- EP2497464
- WO2011160798

### Non patent documents

- Amr R. Ibrahim et al., 'Poor adherence is the main reason for loss of CCyR and imatinib failure for chronic myeloid leukemia patients on long-term therapy' Blood, 2011 vol. 117, no. 14, pgs. 3733-3736.
- European Pharmacopoeia 7.0 04/2010:1502

## Claims

1. An immediate release oral pharmaceutical composition consisting of imatinib mesylate in an amount equivalent to 600 mg of imatinib free base and at least one pharmaceutically acceptable excipient, wherein the composition is in the form of a film-coated tablet comprising from 95% to 99% by weight of imatinib mesylate salt based on the total weight of the film-coated Tablet wherein the pharmaceutically acceptable excipients comprised in the tablet core are selected from the group consisting of diluents, glidants, lubricants, and combinations thereof wherein:
diluents are selected from the group consisting of lactose, calcium carbonate, calcium sulfate, sugar, dextrates, dextrin, dextrose, dibasic calcium phosphate, kaolin, magnesium carbonate, magnesium oxide, maltodextrin, mannitol, polymethacrylates, potassium chloride, sodium chloride, sorbitol, talc, and combinations thereof;
Glidants are selected from the group consisting of magnesium trisilicate, talc, and combinations thereof; and
lubricants/lubricating agents are selected from the group consisting of magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, zinc stearate, and combinations thereof.

2. The pharmaceutical composition according to claim 1 **characterised in that** it comprises 96.77% to 99% by weight of imatinib mesylate salt based on the total weight of the film-coated tablet.

3. The pharmaceutical composition according to any of the preceding claims **characterised in that** the tablet core consists of imatinib mesylate and one or more lubricants.

4. The pharmaceutical composition according to any of the preceding claims wherein imatinib mesylate is in alpha form, **characterized by** a powder X-ray diffraction (PXRD) pattern having peaks at following 2θ angle positions: about 4.9, 10.5, 14.9, 16.5, 17.7, 18.1, 18.6, 19.1, 21.3, 21.6, 23.2, 24.9, 28.0, and 28.6 ± 0.2 degrees.

5. The pharmaceutical composition according to any of the preceding claims wherein the tablet core is coated with a solution or dispersion in an organic solvent or mixture of organic solvents of a film coating agent comprising hydroxypropylmethyl cellulose.

6. The pharmaceutical composition according to any of the preceding claims wherein the tablet core is manufactured by direct compression.

7. The pharmaceutical composition according to any of the preceding claims consisting of
95%-99%of Imatinib mesylate α-form
0-3% of Lactose monohydrate
0.1%-1% of Magnesium stearate
0.75%-1.25% of a film coating agent comprising hydroxypropylmethyl cellulose based on the total weight of the film coated tablet.

8. The pharmaceutical composition according to any of the preceding claims consisting of 717 mg of imatinib mesylate α-form, 4 mg of magnesium stearate, and 7 mg of film coating agent comprising hydroxypropylmethyl cellulose.

9. The pharmaceutical composition according to any of claims 1-7 consisting of 717 mg of imatinib mesylate α-form, 6 mg of magnesium stearate, and 7 mg of film coating agent comprising hydroxypropylmethyl cellulose.

10. A process for preparing an oral pharmaceutical composition according to any of the preceding claims comprising mixing the active ingredient with at least one pharmaceutically acceptable excipients.

11. The process according to claim 10, wherein the tablet core is prepared by direct compression.

12. The process according to claim 11 which comprises mixing imatinib mesylate with a lubricant agent followed by direct compression of the resulting mixture.

13. The process according to any of claims 10-12, further comprising the step ofcoating the tablet core with a solution or dispersion in an organic solvent or mixture of organic solvents of a film coating agent comprising hydroxypropylmethyl cellulose.

## Patentansprüche

1. Eine sofort freisetzende orale pharmazeutische Zusammensetzung, bestehend aus Imatinibmesylat in einer Menge äquivalent zu 600 mg der freien Base von Imatinib und zumindest einem pharmazeutisch annehmbaren Hilfsstoff, wobei die Zusammensetzung in der Form einer filmbeschichteten Tablette vorliegt, umfassend von 95 Gew.-% bis 99 Gew.-% Imatinib-Mesylatsalz basierend auf dem Gesamtgewicht der filmbeschichteten Tablette, wobei die pharmazeutisch annehmbaren Hilfsstoffe, die in dem Tablettenkern enthalten sind, ausgewählt sind aus der Gruppe bestehend aus Verdünnungsmitteln, Fließregulierungsmitteln, Schmiermitteln und Kombinationen davon, wobei:
- die Verdünnungsmittel ausgewählt sind aus der Gruppe bestehend aus Laktose, Kalziumcarbonat, Kalziumsulfat, Zucker, Dextraten, Dextrin, Dextrose, dibasisches Kalziumphosphat, Kaolin, Magnesiumcarbonat, Magnesiumoxid, Maltodextrin, Mannitol, Polymethacrylate, Kaliumchlorid, Natriumchlorid, Sorbitol, Talkum und Kombinationen davon;
- die Fließregulierungsmittel ausgewählt sind aus der Gruppe bestehend aus Magnesiumtrisilikat, Talkum und Kombinationen davon;
- die Schmiermittel/Lubrikanzien ausgewählt sind aus der Gruppe bestehend aus Magnesiumstearat, Kalziumstearat, Glycerolmonostearat, Glycerolpalmitostearat, hydriertes Rizinusöl, hydriertes Pflanzenöl, Mineralöl, Polyethylenglykol, Natriumbenzoat, Natriumlaurylsulfat, Natriumstearylfumarat, Stearinsäure, Talkum, Zinkstearat und Kombinationen davon.

2. Die pharmazeutische Zusammensetzung nach Anspruch 1, **dadurch gekennzeichnet, dass** die Zusammensetzung 96,77 Gew.-% bis 99 Gew.-% Imatinib-Mesylatsalz umfasst basierend auf dem Gesamtgewicht der filmbeschichteten Tablette.

3. Die pharmazeutische Zusammensetzung nach einem der voranstehenden Ansprüche, **dadurch gekennzeichnet, dass** der Tablettenkern aus Imatinibmesylat und einem oder mehreren Schmiermitteln besteht.

4. Die pharmazeutische Zusammensetzung nach einem der voranstehenden Ansprüche, wobei das Imatinibmesylat in alpha-Form vorliegt, **gekennzeichnet durch** ein Pulver-Röntgen-Diffraktogramm (powder X-ray diffraction, PXRD) mit Peaks bei den folgenden 2-theta-Winkelpositionen von etwa 4,9, 10,5, 14,9, 16,5, 17,7, 18,1, 18,6, 19,1, 21,3, 21,6, 23,2, 24,9, 28,0 und 28,6 ± 0,2 °.

5. Die pharmazeutische Zusammensetzung nach einem der voranstehenden Ansprüche, wobei der Tablettenkern mit einer Lösung oder Dispersion eines Filmbeschichtungsmittels, umfassend Hydroxypropylmethylzellulose, in einem organischen Lösungsmittel oder einem Gemisch von organischen Lösungsmitteln beschichtet ist.

6. Die pharmazeutische Zusammensetzung nach einem der voranstehenden Ansprüche, wobei der Tablettenkern mittels Direktkomprimierung hergestellt ist.

7. Die pharmazeutische Zusammensetzung nach einem der voranstehenden Ansprüche, bestehend aus
- 95% - 99% Imatinibmesylat der alpha-Form
- 0-3% Laktosemonohydrat
- 0,1% - 1% Magnesiumstearat
- 0,75% - 1,25% eines Filmbeschichtungsmittels, umfassed Hydroxypropylmethylzellulose
basierend auf dem Gesamtgewicht der filmbeschichteten Tablette.

8. Die pharmazeutische Zusammensetzung nach einem der voranstehenden Ansprüche, bestehend aus 717 mg Imatinibmesylat der alpha-Form, 4 mg Magnesiumstearat und 7 mg Filmbeschichtungsmittel, umfassend Hydroxypropylmethylzellulose.

9. Die pharmazeutische Zusammensetzung nach einem der Ansprüche 1 bis 7, bestehend aus 717 mg Imatinibmesylat der alpha-Form, 6 mg Magnesiumstearat und 7 mg Filmbeschichtungsmittel, umfassend Hydroxypropylmethylzellulose.

10. Verfahren zur Herstellung einer oralen pharmazeutische Zusammensetzung nach einem der voranstehenden Ansprüche, umfassend das Vermischen des Wirkstoffs mit zumindest einem pharmazeutisch annehmbaren Hilfsstoff.

11. Verfahren nach Anspruch 10, wobei der Tablettenkern mittels Direktkomprimierung hergestellt wird.

12. Verfahren nach Anspruch 11, umfassend das Vermischen von Imatinibmesylat mit einem Fließregulierungsmittel gefolgt von einer Direktkomprimierung des resultierenden Gemisches.

13. Verfahren nach einem der Ansprüche 10 bis 12, ferner umfassend den Schritt des Beschichtens des Tablettenkerns mit einer Lösung oder Dispersion eines Filmbeschichtungsmittels, umfassend Hydroxypropylmethylzellulose, in einem organischen Lösungsmittel oder einem Gemisch von organischen Lösungsmitteln.

## Revendications

1. Composition pharmaceutique orale à libération immédiate constituée de mésylate d'imatinib sous une quantité équivalente à 600 mg de base libre d'imatinib, et d'au moins un excipient pharmaceutiquement acceptable, la composition se présentant sous la forme d'un comprimé pelliculé comprenant de 95% à 99% en poids de sel de mésylate d'imatinib par rapport au poids total du comprimé pelliculé, les excipients pharmaceutiquement acceptables inclus dans le noyau du comprimé étant sélectionnés parmi le groupe constitué des diluants, des agents de glissement, des lubrifiants, et des combinaisons de ceux-ci, dans laquelle :
les diluants sont sélectionnés parmi le groupe constitué du lactose, du carbonate de calcium, du sulfate de calcium, du sucre, des dextrates, de la dextrine, du dextrose, du phosphate dibasique de calcium, du kaolin, du carbonate de magnésium, de l'oxyde de magnésium, de la maltodextrine, du mannitol, des polyméthacrylates, du chlorure de potassium, du chlorure de sodium, du sorbitol, du talc, et des combinaisons de ceux-ci ;
les agents de glissement sont sélectionnées parmi le groupe constitué du trisilicate de magnésium, du talc, et des combinaisons de ceux-ci ; et
les lubrifiants/agents de lubrification sont sélectionnés parmi le groupe constitué du stéarate de magnésium, du stéarate de calcium, du monostéarate de glycéryle, du palmitostéarate de glycéryle, de l'huile de ricin hydrogénée, de l'huile végétale hydrogénée, de l'huile minérale, du polyéthylène-glycol, du benzoate de sodium, du laurylsulfate de sodium, du stéarylfumarate de sodium, de l'acide stéarique, du talc, du stéarate de zinc, et des combinaisons de ceux-ci.

2. Composition pharmaceutique selon la revendication 1, **caractérisée en ce qu'**elle comprend de 96,77% à 99% en poids de sel de mésylate d'imatinib par rapport au poids total du comprimé pelliculé.

3. Composition pharmaceutique selon l'une quelconque des revendications précédentes, **caractérisée en ce que** le noyau du comprimé est constitué du mésylate d'imatinib et d'un ou plusieurs lubrifiants.

4. Composition pharmaceutique selon l'une quelconque des revendications précédentes, dans laquelle le mésylate d'imatinib se présente sous une forme alpha, **caractérisée par** un motif de diffraction des rayons X sur poudre (PXRD) présentant des pics dans les positions d'angle 2θ suivantes : environ 4,9, 10,5, 14,9, 16,5, 17,7, 18,1, 18,6, 19,1, 21,3, 21,6, 23,2, 24,9, 28,0 et 28,6 ± 0,2 degrés.

5. Composition pharmaceutique selon l'une quelconque des revendications précédentes, dans laquelle le noyau du comprimé est enrobé dans une solution ou dispersion, en solvant organique ou en mélange de solvants organiques, d'un agent d'enrobage comprenant de l'hydroxypropylméthyl-cellulose.

6. Composition pharmaceutique selon l'une quelconque des revendications précédentes, dans laquelle le noyau du comprimé est fabriqué par compression directe.

7. Composition pharmaceutique selon l'une quelconque des revendications précédentes, comprenant :
de 95% à 99% de forme α de mésylate d'imatinib ;
de 0 à 3% de monohydrate de lactose ;
de 0,1% à 1% de stéarate de magnésium ;
de 0,75% à 1,25% d'un agent d'enrobage comprenant de l'hydroxypropylméthyl-cellulose,
par rapport au poids total du comprimé pelliculé.

8. Composition pharmaceutique selon l'une quelconque des revendications précédentes, comprenant 717 mg de forme α de mésylate d'imatinib, 4 mg de stéarate de magnésium, et 7 mg d'agent d'enrobage comprenant de l'hydroxypropylméthyl-cellulose.

9. Composition pharmaceutique selon l'une quelconque des revendications 1 à 7, comprenant 717 mg de forme α de mésylate d'imatinib, 6 mg de stéarate de magnésium, et 7 mg d'agent d'enrobage comprenant de l'hydroxypropylméthyl-cellulose.

10. Procédé pour préparer une composition pharmaceutique orale selon l'une quelconque des revendications précédentes, comprenant le mélange de l'ingrédient actif avec au moins un excipient pharmaceutiquement acceptable.

11. Procédé selon la revendication 10, selon lequel le noyau du comprimé est préparé par compression directe.

12. Procédé selon la revendication 11, qui comprend le mélange de mésylate d'imatinib avec un agent lubrifiant, suivi par une compression directe du mélange résultant.

13. Procédé selon l'une quelconque des revendications 10 à 12, comprenant de plus l'étape d'enrobage du noyau du comprimé par une solution ou dispersion, en solvant organique ou en mélange de solvants organiques, d'un agent d'enrobage comprenant de l'hydroxypropylméthyl-cellulose.
